Europäisches Patentamt

European Patent Office

Office européen des brevets

(19)

(11) **EP 0 712 623 B1**

(12) **EUROPEAN PATENT SPECIFICATION**

(45) Date of publication and mention
of the grant of the patent:
**17.01.2001 Bulletin 2001/03**

(51) Int Cl.7: **A61K 7/09**

(21) Application number: **95308241.9**

(22) Date of filing: **17.11.1995**

(54) **Permanent waving composition**

Dauerwellenzusammensetzung

Composition pour onduler les cheveux

(84) Designated Contracting States:
**DE FR GB**

(30) Priority: **17.11.1994 JP 28382894**
**28.06.1995 JP 16271395**

(43) Date of publication of application:
**22.05.1996 Bulletin 1996/21**

(73) Proprietor: **SHISEIDO COMPANY LIMITED**
**Chuo-ku Tokyo (JP)**

(72) Inventors:
• **Kubo, Sanae**
**Yokohama-shi, Kanagawa-ken (JP)**

• **Arai, Yasuhiro**
**Yokohama-shi, Kanagawa-ken (JP)**
• **Kawata, Emiko**
**Yokohama-shi, Kanagawa-ken (JP)**

(74) Representative: **Woods, Geoffrey Corlett**
**J.A. KEMP & CO.**
**Gray's Inn**
**14 South Square**
**London WC1R 5LX (GB)**

(56) References cited:
**EP-A- 0 288 151**    **FR-A- 1 072 866**
**FR-A- 1 241 160**    **GB-A- 889 572**

## Description

This invention relates to the art of permanent waving of hair and, more particularly, to novel compositions which impart substantially improved curl characteristics to the hair while also virtually eliminating the objectionable odor typically associated with permanent waving.

The permanent waving of hair is a well established and well developed art in which substantial attention has been directed to improve the present level of technology. Although substantial changes have occurred throughout the last decades, various problems continue to plague the industry in spite of numerous attempts to reduce or eliminate these problems.

In order to best understand the present state of the art and the problems existing therein, it is important to reiterate that hair fibers are composed of a unique protein called "keratin" which is distinguished by the fact that it contains a very significant amount of an amino acid, cystine, which contains the element sulfur in addition to the elements nitrogen, oxygen, carbon and hydrogen. In the natural synthesis of hair, the element sulfur covalently links intra or inter polypeptide chains (K) through two sulfur atoms (S-S) to give keratin protein (K-S-S-K). Only by chemical action can thick covalent linkage be broken.

When a sufficient number of hair disulfide bonds have been broken, the hair is realigned to pair previously unpaired hair protein thiol groups opposite each other. At this point, the hair is rinsed, removing the unreacted thiol waving agent and any water soluble disulfide reaction product formed from it. Then, the hair is saturated with an oxidizing agent, or neutralizer, such as hydrogen peroxide, bromate salt, or chlorite salt to reform disulfide bonds between the newly paired hair protein thiols, thereby giving the hair a new configuration or wave, or adding curl to the hair. By rebonding the sites of the reduced keratin in their new curled configuration, a permanent set which is impervious to water is established.

The most commonly used reducing agents employed in the permanent deformation of hair keratin are salts and esters of thioglycolic acid. Other reducing agents include cysteine, cysteamine, thiolactic acid and their derivatives. These reducing agents are very effective in the reduction of disulfide bonds and under certain conditions can reduce more than 50% of the keratin cystine bonds.

Although effective in providing excellent reducing capabilities, the above mercaptans and their corresponding derivatives possess problems that are difficult to control. One of the disadvantages is the fact that permanent waving must typically be conducted at elevated pH levels. However, since the skin surface of humans is usually at a weak acidic level, the use of products at an elevated pH causes discomfort, irritation, and/or adverse reactions. Although low pH levels would be desirable, the permanent wave results produced at lower pH levels are not satisfactory. Other disadvantages of prior art reducing agents are the irreversible fiber alteration, as made evident by increased fiber porosity and decreased mechanical tensile properties, and malodor.

Much efforts have been expended in attempts to minimize these attributes. These include pretreatments, barriers which decrease the rate of diffusion, reduction of the mercaptan concentration and/or the pH of the reducing agents, and duration of reduction time. Many of these pretreatments yield other undesirable characteristics such as oily, greasy, and dirty feeling of the hair fiber.

Although substantial effort has occurred in prior art permanent wave compositions, these prior art compositions have been incapable of eliminating or substantially reducing the malodor resulting from the permanent wave procedure. Although various prior art attempts have been made, no successful or elimination or substantial reduction of this malodor has been attained.

Furthermore, in the art of permanent waving, there is much trial and error, with the hair being over-processed, in some instances. The characteristics of over-processing are raspy feel to the hair or a loss of the natural underlying color. Structural evaluation of the hair fiber by instrumentation usually reveals that the structural integrity of the hair is lessened, which is evidenced by either an increase in the amount of cysteine and cysteic acid or a lessening of the cystine content relative to the hair not so processed.

For example, in Japanese Laid-open Patent Application No. 69717/1986, it is proposed to avoid the use of thioglycolic acid as a reducing agent and use thiolactic acid instead for the purpose of reducing a bad smell, and use hydrolyzates of certain natural proteins or derivatives of the hydrolyzates in combinations for the purpose of reducing the damage of hair. As stated above, according to the above official bulletin, result in some degree is obtained according to the method of evaluating the damage of hair based on the amount of cysteic acid in the treated hair.

These polymer treatments have had temporary effect on promoting cohesion and decreasing or retarding the rate of water uptake by the hair fiber, while other treatments have attained temporary improvement of such physical characteristics as seen, manageability and strength. However, these prior art conditioning agents merely provide a temporary benefit and are incapable of satisfying the long-felt need for substantially permanent hair condition improvement.

Furthermore, permanent change in hair keratin coupled with operator error, provides inevitable damage to the hair fibers. This damage is measured by evaluating the tensile strength of hair keratin fibers caused by these chemical treatments. Therefore, it would be advantageous to provide treatments that would produce results of a permanent nature and minimum damage to hair keratin.

Therefore, it is a principal object of the present invention to provide a composition for permanently waving hair fibers which is capable of imparting to the head of hair a durable, long-lasting permanent hair set retention, while substantially reducing the malodor typically associated with permanently waved hair.

Another object of the present invention is to provide a permanent wave composition having the characteristic features described above which is capable of conditioning the hair fibers and improving physical properties of the treated hair such as shine, luster, softness, manageability, hair body, and thickness.

Another object of the present invention is to provide a permanent wave composition having the characteristic features described above which is capable of imparting a long-lasting permanent wave or setting property to the hair, while substantially reducing hair damage caused during the reduction and oxidation processes.

A further object of the present invention is to provide a permanent wave composition having the characteristic features described above which is capable of improving the elastic and tensile properties of the hair fibers.

Other and more specific objects will in part be obvious and will in part appear hereinafter.

To one's surprise, the present inventors found that the above object can be accomplished when an aqueous hair reducing or permanent waving composition for use in permanent waving of hair contains as a reducing agent thiolactic acid or a salt thereof with a particular base and contains the base as an alkali agent, and the ratio of the particular base moiety to the thiolactate moiety is selected so as to fall within a particular range, without a necessity that the composition contains a protein hydrolyzate as described in the above Japanese Laid-open Patent Publication No. 69717/1986. Namely, particularly, it was realized that it is possible to provide a permanent waving composition whereby not only the damage of hair due to ""over-process" can be prevented from progressing, but permanent wave further excellent in permanency can be obtained easily.

Thus, according to the present invention, there is provided an aqueous hair reducing or permanent waving composition for use in permanent waving of hair, containing a reducing agent and an alkali agent, said composition being characterized in that

A. the composition contains as the reducing agent thiolactic acid in the free form, or thiolactic acid in the form of a salt with at least one base selected from the group consisting of ammonia, monoethanolamine and isopropanolamine, and contains as the alkali agent at least one basic compound which is selected from the group consisting of ammonium hydroxide, monoethanolamine and isopropanolamine, and is forming thiolactic acid in the above salt form or exists without forming such a salt, and

B. the molar ratio of the total monoethanolamine and/or isopropanolamine moiety to the total thiolactate moiety in the composition is between 0.3 to 1.0.

As understood from the ingredients constituting the composition, the permanent waving composition provided by the invention is directed to a so-called permanent wave primary agent used at the reduction step before the oxidation treatment in which the disulfide bond of the keratin protein is re-formed with a neutralizer and fixed.

Particularly, the composition of the invention is characterized by containing, based the molar concentration of the total thiolactate moiety from thiolactic acid or its salt contained as a reducing agent in the composition, the total monoethanolamine and/or isopropanolamine moiety which are bases added as alkali agents, in the range shown in the following formula:

$$0.3 \leqq \frac{\Sigma([MEA^{+}] + [MEA] + [IPA^{+}] + [IPA])}{\Sigma[TL^{-}]} \leqq 1.0$$

In the formula, $\Sigma[TL^{-}]$ represents the molar concentration of the total thiolactate moiety contained in the composition, namely the total amount of the thiolactate moieties from the free thiolactic acid and thiolactic acid salt(s);

[MEA$^{+}$] and [IPA$^{+}$] represent the molar concentrations of the amine moieties of monoethanolamine thiolactate and isopropanolamine thiolactate, respectively, when they exist in the composition;

[MEA] and [IPA] represent the molar concentrations of free monoethanolamine and free isopropanolamine, respectively, when they exist in the composition; and $\Sigma$ represents the total of those molar concentrations. The monoethanolamine and isopropanolamine moiety conveniently consists of monoethanolamine alone, but may consist of isopropanolamine alone or a mixture of monoethanolamine and isopropanolamine.

Further, the molar concentration is preferably between 0.5 and 1.0.

In this preferred range, optimum waving effect is achieved, with less damage being caused to the hair. Furthermore, substantially less malodor is found at this preferred level, along with producing a smooth touch to the hair after perming.

It has also been found that whenever the molar ratio of the monoethanolamine and/or isopropanolamine moieties to the thiolactale moiety exceeds 1.0, beauticians often experience rough hands after repeated use. Furthermore, at molar ratios lower than 0.3, discomfort on the skin surface is experienced by beauticians, as well as a strong malodor and a reduced waving effect.

Thiolactic acid used in the invention may be any one kind of stereoisomer, or may be a mixture thereof. In order to efficiently use the composition at the permanent waving step of hair, the total molar concentration of the thiolactic acid salt(s) and thiolactic acid (if any) contained in the composition is set between 0.3 M and 2.2 M.

Particularly as the cold two-bath permanent wave primary agent, when it is intended to provide a permanent waving composition for normal hair, it is preferred to adopt the concentration of thiolactic acid or its salt between 0.4 M and 1.1 M, and when it is intended to provide a permanent waving composition for previously damaged hair, it is preferred to adopt the concentration of thiolactic acid or its salt between about 0.4 M and 0.8 M.

Alternatively, as an exothermic two-bath permanent wave primary agent for preparation at the time of use, 1.2 to 2.2 M is appropriate. The concentration is fairly high compared with the cold two-bath type, but the reason is that since part of the agent is mixed with an aqueous hydrogen peroxide solution at the time of use and thereby thiolactic acid and its salt are converted to dithiodilactic acid, and the total volume is increased and relatively diluted, it is necessary to heighten the concentration of thiolactic acid.

The permanent waving composition according to the present invention can be employed to provide an automatic stop effect against over process when the desired level of permanent waving has been attained. In order to assure that the permanent wave lotion possesses this automatic stop, it has been found that the total quantity of thiolactic acid or its salts should range between about 1.2 and 2.2 M.

The total amount of bases existing in a free state in the composition is not limited because a preferred amount thereof varies depending on the kind of base to be used, but usually, is adjusted so as to fall within the range of 0.2 N (normality) and 0.8 N, more preferably within the range of 0.4 N and 0.7 N. When it is above 0.8 N, there is a tendency that the damage of hair preferentially occurs and at the same time a load on the skin of the subjects is increased.

In a preferred embodiment based on another observation, the composition according to the invention is set at a pH between 7.5 and 9.5. A further preferred pH value is between 8.5 and 9.2, and when a desired pH value is not attained by the reducing agent and the alkali agent, other alkali agents than the above-mentioned, for example, at least one of ammonium carbonate, ammonium bicarbonate, sodium carbonate, guanidine, lysine, arginine, 2-amino-2-methylpropanol, sodium hydroxide, etc. can be added in a small amount.

In one preferred embodiment of the present invention, monoethanolamine thiolactate and ammonium thiolactate are employed as the reducing agents or thiol compounds of the permanent waving composition. In this regard, it has been found that the preferred composition comprises a molar ratio of monoethanol and/or isopropanolamine thiolactates to ammonium thiolactate of 0.25 : 0.75 ~ 1.0 : 0.0.

Although the thiol compounds or reducing agents and alkalizing agents detailed above can be employed in combination or individually, providing a wide variety of various formulations coming within the scope of the present invention, the easiest and most economical formulation to manufacture comprises monoethanolamine thiolactate and ammonia thiolactate. However, any other combination can be employed with equal efficacy.

In addition to employing the thiol compounds and alkalizing agents detailed above, the permanent waving composition according to the present invention may also incorporate additional additives. One additive, which may be employed in order to reduce the cost of the permanent waving composition, comprises other thiol compounds chosen from thioglycolic acid, ammonium thioglycolate, monoethanolamine thioglycolate, cysteine and salts thereof and N-acetyl cysteine. Also included are oxidants or oxidised derivatives of these compounds. If additional thiol compounds selected from this group are employed in the permanent waving composition, it has been found that the mole content should range between about 0.1 and 0.8.

Additional agents that can be employed in the permanent waving composition of the present invention include swelling penetrating, wetting, chelating, and conditioning agents, protein hydrolysates, preservatives, and surfactants. In particular, one or more agents selected from the group consisting of urea, alkyl urea, guanidine, guanidine chloride, phenoxyethanol, phenoxypropanol, benzyl alcohol, lauryl betaine, cationic, nonionic and anionic surfactants have been found to be particularly useful in improving the overall waving effect provided by the permanent wave composition of the present invention. The inclusion of one or more additives selected from this group has been found to reduce the processing time required for attaining the desired permanent wave, as well as adjusting the waving effect. In order to attain these enhanced benefits, the total quantity of these ingredients preferably ranges between about 0.05% and 5% by weight based upon the weight of the entire composition.

The efficacy of the present invention is clearly detailed herein, along with the ability of the composition according to the present invention to permanently wave hair with substantially improved, long-lasting physical enhancements and characteristics being attained. In addition, improved comfort to the beautician is provided, both in skin reaction and in the reduction of the malodor typically associated with permanent wave compositions.

The following examples are presented in order to demonstrate the efficacy of a permanent waving composition according to the present invention.

In order to prove the efficacy of the present invention, numerous hair tresses were tested by permanent waving, using permanent wave compositions manufactured in accordance with the present invention and comparing these results to hair tresses permanently waved with generally conventional reducing agents. Each sample tested was evaluated as to its waving effect, its damage to the hair, the malodor produced, and its safety to the skin.

Waving Efficiency

In determining and evaluating the waving efficiency or tightness, numerous samples of twelve freshly shampooed normal hair fibers were knotted at the root end and cut to a length of 114 mL from the knot. The bundle was then wound around a glass rod having a diameter of 7.0 mm. Using $80\,\mu L$, the aqueous permanent waving composition was evenly applied to the hair fibers for over two minutes, after which the wrapped rods were placed in a container, closed, and maintained at a constant temperature of about 37°C for 20 minutes. Once completed, the hair fibers were rinsed with running water for 1 minute.

After rinsing, the hair fibers were immersed in 5 mL of a neutralizer consisting of 6% sodium bromate, at a pH of 6, adjusted by phosphoric acid. Any excess neutralizer was removed and the hair fibers were allowed to process with the lotion for 10 minutes in a water tank of 37°C. Thereafter, the hair fibers were washed in water and removed from the glass rods. Then, both the length of the hair fiber and the diameter of the resulting curl were recorded.

Generally, the smaller the diameter of the hair curl, the stronger the waving action of the permanent wave lotion, with 7 mm being the smallest diameter. Similarly, the shorter the length of the coil, the more elastic is the permanent wave lotion, with 5 mm being the shortest and 114 mm being the longest.

Damage Occurring to the Hair Fibers

In order to evaluate the damage occurring to the hair fibers, the hair fibers tested were dried overnight at room temperature and weighed on a balance in a controlled atmosphere of 65% relative humidity and 23°C. The hair fibers were then dipped in deionized water for one hour and excess water was removed using a centrifuge, operating at 2,000 rpm for two minutes. Thereafter, the fibers were immediately weighed. The rate of moisture absorbency of the hair fiber was calculated using the following formula:

$$\text{Moisture Absorbency Rate(\%)} = \frac{\text{wet hair weight - dry hair weight}}{\text{dry hair weight}} \times 100$$

As is well known, the processing of hair can often cause damage to the hair fiber by making the fiber or hair keratin more porous. Consequently, the more water absorbed by the hair fiber, the more damage that has been caused to the hair. By comparing the moisture absorbent rate, the more damage caused to the hair fiber can be determined.

Additional tests concerning hair damage was also done by observations. In this regard, five individuals evaluated the touch of the hair by hand after natural drying. In conducting this evaluation, the following rating schedule was employed:

| Rating | Condition |
|---|---|
| Excellent | smooth and very good |
| Fair | good |
| Poor | rough or not smooth |
| Bad | not smooth, disturbed waves, and slightly discolored. |

Malodor

In order to determine the quantity of ammonia emanating from the permanent waving compositions, which is a contributing factor to the malodor associated with the composition, 10 mL of each permanent waving composition tested was individually placed into a 600 mL glass container. The glass container was closed and left for ten minutes in a water tank maintained at 37°C . Thereafter, the gas found in the head space or upper zone of the container was analyzed using an ammonia detector. The larger the value provided by the ammonia detector, the greater the quantity of ammonia found in the head space of the container.

Similarly, the hydrogen sulphide content of each of the permanent wave compositions tested was also evaluated. In conducting these tests, one gram of hair and 10 mL of the aqueous permanent waving composition was placed in a 600 mL glass container. The container was closed and left for 20 minutes in a water tank of 37°C. Thereafter, the container was opened and, using a hydrogen sulphide detector, a 100 mL sample of the gas in the head space of the container was taken and analyzed. Using a hydrogen sulphide sensor manufactured by Matheson Gas Products, the amount of hydrogen sulphide contained within the 100 mL sample was determined and recorded. These values were compared. The larger value represents the stronger or greater quantity of hydrogen sulphide, which would lead to an increase in malodor.

As a further determination of the malodor present with each permanent waving composition, individual odor testing was also conducted. In this regard, five individuals were asked to smell each of the samples after the hydrogen sulphide test was conducted. Each individual was then asked to rate each permanent wave lotion, based upon the following criteria:

| Rating | Condition |
|---|---|
| Excellent | much milder than other general perm agents |
| Fair | mild |
| Poor | ammonia smell is strongly felt |
| Bad | ammonia smell is too strong and pain is felt in the nose. |

Safety

The final test conducted on each of the aqueous permanent waving compositions was a determination of the skin reaction each permanent wave composition produced on individuals. In conducting these tests, four volunteers were used, with each volunteer duplicating the same test procedures. For each aqueous permanent waving composition, a number of drops were placed on both hands of each individual, in various locations, and left in position for 20 minutes. During the time period, each individual was asked questions about the irritation, if any, being caused. These tests were given three separate times for each permanent wave composition, with the tests being conducted every other day. The results were then evaluated to determine rough hands, dryness, and skin surface effects. Using the following criteria, the results were quantified:

| Rating | Condition |
|---|---|
| Irritation | |
| ++ | strong irritation, and pain (particularly felt at the second and eighth time) |
| + | moderate pain |
| - | no pain or irritation |
| Rough Hands | |
| +++ | the front skin is dry and whitish, and partially stripped off at corners |
| ++ | the front skin is dry and whitish |
| + | the front skin is partially whitish (at skin pores) |
| - | normal |

Examples 1-9

In order to demonstrate the efficacy of the aqueous permanent waving compositions according to the present invention, numerous tests were conducted on various permanent wave formulations made in accordance with the present invention, as well as on control or comparative formulations used for comparative purposes. In Table I, the detailed formulations for a first group of such permanent wave compositions is provided.

## TABLE I

### Permanent Wave compositions

| Ingredients (% by Weight) | EXAMPLES | | | | | | | | |
|---|---|---|---|---|---|---|---|---|---|
| | 1 (Control 1) | 2 (Control 2) | 3 | 4 | 5 | 6 | 7 | 8 (Comparison) | 9 (Comparison) |
| Ammonium Thioglycolate (containing 60% of thioglycolic acid) | 15.35 | | | | | | | | |
| Ammonium Thiolactate (containing 60% of thiolactic acid) | | 17.68 | 15.47 | 13.26 | 8.8 | 4.42 | | | |
| Monoethanolamine Thiolactate (containing 50% of thiolactic acid) | | | 2.66 | 5.30 | 10.6 | 15.92 | 21.22 | 21.22 | 21.22 |
| Monoethanolamine | 1.52 | 1.52 | 1.52 | 1.52 | 1.52 | 1.52 | | 0.76 | 1.52 |
| Ammonium (28%) | 0.93 | 0.93 | 0.93 | 0.93 | 0.93 | 0.93 | 2.45 | 1.69 | 0.93 |
| Ammonium Carbonate | 0.40 | 0.40 | 0.40 | 0.40 | 0.40 | 0.40 | 0.40 | 0.40 | 0.40 |
| EDTA | 0.05 | 0.05 | 0.05 | 0.05 | 0.05 | 0.05 | 0.05 | 0.05 | 0.05 |
| Nonoxynol-15 | 2.00 | 2.00 | 2.00 | 2.00 | 2.00 | 2.00 | 2.00 | 2.00 | 2.00 |
| Deionized Water | qs to 100% | qs to 100% | qs to 100% | qs to 100% | qs to 100% | qs to 100% | qs to 100% | qs to 100% | qs to 100% |

After preparing these aqueous permanent wave compositions, each of the aqueous permanent wave compositions were evaluated for their efficacy, using the procedures detailed above. In Table II, the results attained from the evaluations are provided.

## TABLE II

### Permanent Wave Evaluations

| | EXAMPLES | | | | | | | | |
|---|---|---|---|---|---|---|---|---|---|
| | 1 (control 1) | 2 (Control 2) | 3 | 4 | 5 | 6 | 7 | 8 (Comparison) | 9 (Comparison) |
| Molar Ratio: (Monoethanolamine moieties) / (Thiolactate moieties) | Ammonium Thioglycolate | 0.24 | 0.36 | 0.49 | 0.74 | 1.00 | 1.00 | 1.12 | 1.25 |
| Molar Ratio: (Monoethanolamine Thiolactate) / (Thiolactate) | | 0.00 | 0.13 | 0.25 | 0.50 | 0.75 | 1.00 | 1.00 | 1.00 |
| Wave Effect – | | | | | | | | | |
| Strength (Diameter of Curl) | 13.4 | 13.2 | 11.1 | 10.8 | 10.5 | 11.0 | 11.8 | 13.0 | 13.7 |
| Elasticity (Length of Curl) | 61.1 | 55.8 | 51.3 | 50.9 | 48.3 | 50.2 | 51.7 | 57.7 | 58.3 |
| Damage – | | | | | | | | | |
| Moisture Absorbency | 38.5 | 36.0 | 33.90 | 34.0 | 32.5 | 29.6 | 27.8 | 28.3 | 28.1 |
| Touch | Bad | Poor | Fair | Fair | Excellent | Excellent | Excellent | Fair | Fair |

EP 0 712 623 B1

## TABLE II (continued)

| | EXAMPLES | | | | | | | | |
|---|---|---|---|---|---|---|---|---|---|
| | 1 (control 1) | 2 (Control 2) | 3 | 4 | 5 | 6 | 7 | 8 (Comparison) | 9 (Comparison) |
| Odor – | | | | | | | | | |
| Ammonia (ppm) | 12000 | 9000 | 6800 | 5800 | 3200 | 2000 | 2000 | 1800 | 1500 |
| Hydrogen Sulphide (ppm) | 280 | 61 | 59 | 60 | 65 | 61 | 64 | 64 | 62 |
| Odor Evaluation | Bad | Bad | Fair | Fair | Fair | Excellent | Excellent | Fair | Fair |
| Safety – | | | | | | | | | |
| Irritation | ++ | + | +/- | +/- | - | - | - | - | - |
| Roughness | ++ | + | - | - | - | - | - | ++ | +++ |

As is evident from a review of Table II, permanent wave compositions made in accordance with the present invention (Examples 3-7) demonstrate a substantially improved waving efficiency and hair curl elasticity than was found with the control and comparative samples of Examples 1, 2, 8, and 9. With regard to hair damage, the aqueous permanent wave composition of the present invention demonstrated a substantially lower moisture absorption rate, as well as being milder to the touch. This is particularly evident in comparing the compositions according to the present invention with control Examples 1 and 2.

Similarly, the aqueous permanent waving compositions according to the present invention has shown substantially improved results in reducing the malodor typically found with permanent waving. In addition, substantially less irritation was caused to the hands using the permanent wave lotions of the present invention. Based upon the foregoing test evaluations, it is apparent that the aqueous permanent waving compositions detailed in Examples 3-7 are substantially superior to the control and comparative samples detailed in Examples 1, 2, 8 and 9.

Examples 10-15

In Table III, test formulations for additional permanent wave compositions are provided. In this group, aqueous permanent wave compositions made in accordance with this invention (Examples 10 and 11) were prepared and evaluated against aqueous permanent wave compositions formulated with thiol compounds incorporating non-smelling organic alkali agents (Comparative Examples 12-15). Using the same procedures detailed above, these compositions were similarly tested and evaluated. In Table IV, the results attained from evaluating Examples 10-15 are provided.

TABLE III

Permanent Wave Compositions

| Ingredient (% by Weight) | EXAMPLES | | | | | |
|---|---|---|---|---|---|---|
| | 10 | 11 | 12 (Comparison) | 13 (Comparison) | 14 (Comparison) | 15 (Comparison) |
| Monoethanolamine Thiolactate (containing 30% of thiolactic acid) | 17.68 | | | | | |
| Isopropanolamine Thiolactate (containing 30% of thiolactic acid) | | 17.68 | | | | |
| 2-Amino-2-Methylpropanol Thiolactate (containing 30% of thiolactic acid) | | | 17.68 | | | |
| Lysine Thiolactate (containing 30% of thiolactic acid) | | | | 17.68 | | |
| Arginine Thiolactate (containing 30% of thiolactic acid) | | | | | 17.68 | |
| Triethanolamine Thiolactate (containing 30% of thiolactic acid) | | | | | | 17.68 |
| Ammonium Thiolactate | 10.62 | 10.62 | 10.62 | 10.62 | 10.62 | 10.62 |
| Monoethanolamine | 1.52 | 1.52 | 1.52 | 1.52 | 1.52 | 1.52 |
| Ammonia (28%) | 0.93 | 0.93 | 0.93 | 0.93 | 0.93 | 0.93 |
| Ammonium BiCarbonate | proper dose | proper dose | proper dose | proper dose | proper dose | proper dose |
| ETDA | 0.05 | 0.05 | 0.05 | 0.05 | 0.05 | 0.05 |
| Nonoxynol-15 | 2.00 | 2.00 | 2.00 | 2.00 | 2.00 | 2.00 |
| Deionized Water | qs to 100% | qs to 100% | qs to 100% | qs to 100% | qs to 100% | qs to 100% |

EP 0 712 623 B1

TABLE IV

Analysis

| Evaluation | EXAMPLES | | | | | |
|---|---|---|---|---|---|---|
| | 10 | 11 | 12 (Comparison) | 13 (Comparison) | 14 (Comparison) | 15 (Comparison) |
| Molar Ratio: (Alkylnolamine moieties*) / (Thiolactate moieties) | 0.68 | 0.68 | 0.68 | 0.68 | 0.68 | 0.68 |
| Wave Effect | | | | | | |
| Strength (Length of Curl) | 10.3 | 11.0 | 13.2 | 15.5 | 14.8 | 23.5 |
| Elasticity (Length of Curl) | 48.5 | 51.3 | 63.5 | 63.8 | 65.3 | 77.7 |

\* Alkylnolamine moieties = Monoethanolamine moieties + Isopropanolamine moieties + 2-Amino-2-Methylpropanol moieties + Lysine moieties + Arginine moieties + Triethanolamine moieties

As is evident from a review of Table IV, the permanent wave compositions detailed as Examples 10 and 11 (invention) demonstrated substantially stronger wave effects when compared to Comparative Examples 12-15. Based upon this analysis, it is evident that the incorporation of monoethanolamine lactate or isopropanolamine lactate provides substantially greater hair waving efficacy and lower odor than the other alkalizing agents.

Example 16

In order to further demonstrate the efficacy of the present invention, one preferred formulation of the aqueous permanent composition according to the present invention was prepared and tested. In this experiment, the permanent wave formulation detailed in Table V was employed as representative of an aqueous permanent wave composition for normal hair.

TABLE V

| Permanent Wave Composition for Normal Hair | |
|---|---|
| Ingredients | % by Weight |
| Ammonium Thiolactate Solution (containing 60% of thiolactic acid) | 5.57 |
| Monoethanolamine Thiolactate (containing 40% of thiolactic acid) | 10.22 |
| Monoethanolamine (99%) | 1.21 |
| Ammonia (28% - 30%) | 0.74 |
| Ammonium Bicarbonate | 0.32 |
| EDTA | 0.05 |
| Lauryl Dimethylamine acetate betaine | 0.50 |
| Polyoxyethylene Lauryl Ester | 2.00 |
| Fragrance | 0.50 |
| Phenoxypropanol | 0.50 |
| Amodimethicone[*1] | 1.00 |
| Deionized Water | q.s. to 100% |

*1: Obtained from Toray - Dow Corning Corp.

In preparing this composition, the first seven ingredients listed in Table V were added to 60 parts deionized water and melted thoroughly. Thereafter, the 8th, 9th, and 10th ingredients were separately heated and melted then added into the formulation at a temperature of about 40°C. Finally, ingredient #11 was added along with additional deionized water to form 100%.

Once completed, the composition was tested and analyzed. Table VI provides the analysis resulting for this embodiment of the permanent wave composition.

TABLE VI

| Analysis of Composition | |
|---|---|
|  |  |
| Quantity of thiolactic acid | 0.698M |
| Quantity of free alkali | 0.35N |
| Quantity of monoethanolamine | 0.580M |
| pH (25°C) | 9.1 |
| Molar Ratio: (monethanolamine moieties) / (thiolactate moieties) | 0.831 |
| Molar Ratio: (monoethanolamine thiolactate) / (thiolactate) | 0.55 |
| Quantity of ammonia in headspace | 1900 ppm |
| Quantity of Hydrogen Sulfide in headspace | 32 ppm |

In order to clearly and unequivocally demonstrate the safety of the composition detailed in Table V, an independent testing firm in the United States was employed to conduct safety tests on this permanent wave composition utilizing semi-occlusive tests on 102 individuals.

One hundred of the 102 individuals showed no negative effects from the aqueous permanent waving composition. One person demonstrated arrythmia after 72 hours, while a second person exhibited arrythmia after 72 hours, while a second person exhibited arrythmia after 96 hours. It was concluded that the particularly low level of reaction, ranging between about 1% to 2%, demonstrated an extremely low reaction rate to the aqueous permanent wave composition.

In addition, the aqueous permanent wave composition detailed in Table V was tested for shelf life at different temperatures. In conducting this text, samples, each containing 100 mL of the product, were individually packed and sealed in plastic containers. The containers were stored for six months at 0°C, room temperature, and 40°C. At the end of the six months, each of the samples were tested.

The test results conducted showed that at 40°C, the permanent wave lotion experienced a relative 4% reduction in the concentration of thiolactic acid. The quantity of free alkali and the pH level remained within conventional ranges in all samples.

In a final test, the efficacy of the aqueous permanent wave composition was evaluated using half-head tests. In these tests, six individuals were selected, each of which had normal hair. Half of the head of hair of each individual was permanently waved using the aqueous permanent wave composition defined in Table V, while the other half of the head of hair was permanently waved using a commercially available aqueous ammonium thioglycolate permanent wave composition. The prior art composition employed possessed 0.63 M of thioglycolate acid and a pH of 9.

Both the control composition and the test composition were applied to the hair and allowed to stand for twenty minutes. Following rinsing, the entire head of hair was neutralized using a 6% bromate neutralizer at a pH of 6.5.

The resulting permanently waved heads of hair were evaluated for overall waving effect, hair touch or feel, perm odor, and irritation. In comparing the waving effect attained from these two aqueous permanent wave compositions, all six individuals agreed that the test side was superior in curl and elasticity.

In addition, a follow-up evaluation was made after one week, with each of the test subjects indicating that the test side was noticeably superior to the side permanently waved with the conventional aqueous permanent wave composition.

In overall hair feel or touch, all six individuals agreed that both sides were equally soft to the touch as well as easy to comb after initial perming. However, in the follow-up evaluation conducted one week later, four out of six individuals agreed that the test side showed superior hair feel and combing when wet.

During the permanent waving of each head of hair, each test subject was asked to judge the odor of each of the two aqueous permanent waving compositions. All six individuals agreed that the aqueous permanent wave composition according to the present invention had substantially milder odor than the prior art permanent wave composition. During the follow-up evaluation one week later, three of the six individuals indicated that the side of the head of hair waved with the prior art wave composition had a post-perm odor. This was not perceived on the side with the wave composition of Table V.

In regard to any irritation caused to individuals, neither permanent wave composition was found to result in any skin irritation on the head, face or neck of the individuals. However, two individuals did indicating that during the application process, they felt some skin irritation on the control side. In regard to the beauticians who applied the aqueous permanent waving composition, no rough hands were found from either permanent wave composition.

Based upon the foregoing overall analysis, it is believed that the aqueous permanent wave composition of the present invention is clearly proven to be superior overall to prior art permanent wave compositions.

Example 17

In Table VII, the formulation for an aqueous permanent wave composition is provided which has been found to be particularly effective in providing an automatic stop action when the desired level of permanent waving has been attained. Table VIII provides an overall analysis of the aqueous permanent wave composition detailed in Table VII. In addition to providing a formulation for imparting the auto-stop characteristic to the aqueous permanent wave composition, this permanent wave composition is also particularly useful for hair which is more resistant to permanent waving.

TABLE VII

| Permanent Wave Composition for Resistant Hair | |
|---|---|
| Ingredients | % by Weight |
| Ammonium Thiolactate (containing 60% of thiolactic acid) | 17.48 |
| Monoethanolamine Thiolactate (containing 50% of thiolactic acid) | 20.98 |

TABLE VII (continued)

| Permanent Wave Composition for Resistant Hair | |
| --- | --- |
| Ingredients | % by Weight |
| Monoethanolamine | 3.05 |
| Ammonia (28% - 30%) | 1.02 |
| Ammonium Bicarbonate | 0.12 |
| EDTA | 0.10 |
| Trimethylsilylamodimethicone[*1] | 1.00 |
| Hydrolyzed Wheat Protein | 0.50 |
| Anon BL[*2] | 0.50 |
| Urea | 2.00 |
| Polyoxyethylene (16 E.O.) Nonyl Phenyl Ether | 2.00 |
| Fragrance | 0.50 |
| Azulene (25%) | 0.01 |
| Deionized Water | q.s. to 100mL |

*1: Obtained from Dowcorning Corp.

*2: Lauryl Betaine obtained from Nippon Oil & Fats CO., LTD.

TABLE VIII

| Analysis of Wave Composition | |
| --- | --- |
| Quantity of Thiolactic Acid | 1.95N |
| Quantity of Free Alkali | 0.61N |
| Quantity of Monoethanolamine | 1.49N |
| pH (pH 25°C) | 9.0 |
| Molar Ratio: (Monoethanolamine moieties) / (Thiolactate moieties) | 0.76 |

Using the general procedures detailed above, the aqueous permanent wave composition was applied to hair strands wound on rods and allowed to be processed for fifteen minutes. After rinsing, the neutralizing composition was applied to the hair and allowed to remain for ten minutes.

Once completed, the hair strands were removed from the rods and rinsed in water. The resulting hair fibers exhibited a very elastic wave, and excellent conditioning, easy to comb. Furthermore, the hair fibers exhibited no post-perm odor.

Example 18

By referring to Table IX, another preferred formulation of the permanent wave composition according to the present invention is provided. This formulation is constructed for permanently waving hair fibers which have been previously damaged. In order to prove the efficacy of this invention on damaged hair fibers which were previously tinted were tested. In Table X, the analysis of the aqueous permanent wave composition of Table IX is provided.

TABLE IX

| Permanent Wave Composition for Previously Treated/Damaged Hair | |
| --- | --- |
| Ingredients | % by Weight |
| Ammonium Thiolactate (containing 60% thiolactic acid) | 4.42 |
| Isopropanolamine Thiolactate (containing 40% of thiolactic acid) | 6.63 |

TABLE IX   (continued)

| Permanent Wave Composition for Previously Treated/Damaged Hair | |
|---|---|
| Ingredients | % by Weight |
| Isopropanolamine | 1.00 |
| Cysteine CHI | 1.00 |
| Ammonia (28% - 30%) | 1.00 |
| Ammonium Bicarbonate | proper dose to pH 9.0 |
| Turpinal SL[*1] | 0.50 |
| EMALEX 520[*2] | 1.00 |
| Fragrance | 0.30 |
| Amodimethicone[*3] | 2.00 |
| Deionized Water | q.s. to 100% |

*1: Chelating Agent obtained from Henkel

*2: Polyoxyethylene (20 E. O.) oleylether (Nippon Emulsion CO., LTD.)

*3: Obtained from Toray - Dowcorning Corp.

TABLE X

| Analysis of Wave Composition | |
|---|---|
| Quantity of Thiolactic Acid (HPLC) | 0.48M |
| Quantity of Cysteine (HPLC) | 0.04M |
| Quantity of Isopropanolamine (HPLC) | 0.35M |
| pH (25°C) | 8.80 |
| Molar Ratio: (Isopropanolamine moieties) / (Thoilactate moieties) | 0.78 |
| Quantity of Free Alkali | 0.25N |

In conducting this test, the aqueous permanent wave composition was applied in the manner detailed above in reference to the application of aqueous permanent wave compositions to an actual head of hair. The permanent wave produced from this formulation provided an elastic wave which was easily combed. In addition, the permanent wave was extremely satisfactory and showed only minor discoloration of the previously tinted hair.

Example 19

Further tests were conducted employing the aqueous permanent wave composition in accordance with the present invention as detailed in Table XI. This composition is particularly applicable for use on normal hair. In Table XII, the analysis of the aqueous permanent wave composition of Table XI is provided.

TABLE XI

| Permanent Wave Composition | |
|---|---|
| Ingredients | % by Weight |
| Ammonium Thiolactate (containing 60% thiolactic acid) | 7.10 |
| Monoethanolamine Thiolactate (containing 40% of thiolactic acid) | 7.96 |
| Isopropanolamine Thiolactate (containing 30% of thiolactic acid) | 10.61 |
| Monoethanolamine (99%) | 1.22 |
| Isopropanolamine (99%) | 1.54 |

TABLE XI (continued)

| Permanent Wave Composition | |
|---|---|
| Ingredients | % by Weight |
| Ammonium Bicarbonate | 0.40 |
| EDTA | 0.10 |
| Merquat 100[*1] | 1.00 |
| EMALEX 520[*3] | 2.00 |
| Fragrance | 0.50 |
| Phenoxyethanol | 0.50 |
| Amodimethicone[*2] | 1.00 |
| Deionized Water | q.s. to 100% |

*1: Aqueous Cationic Polymer Solution (Merck Co., Inc.)

*2: Obtained from Toray -Dowcorning Corp.

*3: Polyoxyethylene (20 E.O.) oleylether obtained from NIHON EMULSION CO., LTD.

TABLE XII

| Analysis of Wave Composition | |
|---|---|
| Quantity of Thiolactic Acid | 0.998M |
| Quantity of Free Alkali | 0.47N |
| Quantity of Monoethanolamine | 0.495M |
| Quantity of Isopropanolamine | 0.501M |
| Molar Ratio: (Monoethanolamine moieties) + Isopropanolamine moieties) / (Thiolactate moieties) | 0.998 |
| Molar Ratio: (Thiolactate Monoethanolamine + Thiolactate Isopropanolamine) / (Thiolactate Acid) | 0.60 |
| pH (25°C) | 9.1 |

In testing the aqueous permanent wave composition of this example, the aqueous permanent wave composition was applied to a tress of hair previously wound on a rod as defined above. The aqueous permanent wave composition was allowed to remain on the hair sample for ten minutes and then rinsed for one minute with water. Thereafter, a neutralizing solution was prepared consisting of 2% hydrogen peroxide, using a phosphoric acid buffer and a pH of 4.0. This neutralizing solution was applied to the hair and left on the hair fibers for three minutes.

Thereafter, the hair strands were removed from the rod and rinsed with water. The resulting curl was found to be very elastic with a uniform wave from the scalp to the hair tips. In addition, the hair fibers, when wet or dry, were smooth to the touch, easy to comb, and did not possess any post-perm odor typically found with conventional permanent waves.

Example 20

In order to further demonstrate the efficacy of the present invention, one preferred formulation of the aqueous permanent wave composition according to the present invention was prepared and tested. In this experiment, the permanent wave formulation detailed in Table XIII was employed as representative of an aqueous permanent wave composition.

TABLE XIII

| Permanent Wave Composition | |
|---|---|
| Ingredients | (w/v %) |
| Ammonium Thiolactate (containing 60% thiolactic acid) | 7.96 |
| Monoethanolamine Thiolactate (containing 40% thiolactic acid) | 14.59 |

TABLE XIII   (continued)

| Permanent Wave Composition | |
|---|---|
| Ingredients | (w/v %) |
| Monoethanolamine | 1.73 |
| Ammonium Bicarbonate | 0.70 |
| Ammonia (28% - 30%) | 1.00 |
| Turpinal SL[*1] | 0.10 |
| Anon BL[*2] | 0.80 |
| EMALEX 720[*4] | 0.50 |
| Polymer JR-400[*3] | 0.20 |
| Fragrance | 0.10 |
| Deionized Water | q.s. to 100% |

*1: Chelating Agent obtained from Henkel

*2: Lauryl Betain obtained from Nippon Oil & Fats Co., Ltd.

*3: Cationic cellulose obtained from Union Carbide

*4: Polyoxyethylene (20 E.O.) laulylether obtained from NIHON EMULSION CO., LTD.

In preparing this composition, the first seven ingredients listed in Table XIII were added to 60 parts deionized water and uniformly dissolved. Separately, the 8th ingredient was heated and melted, the 10th ingredient was added at about 40°C and dissolved uniformly, and 10 parts deionized water was added and dissolved uniformly. Further, the 9th ingredient was added to 20 parts deionized water and dissolved uniformly. Then, this solution was mixed with the above solutions, the whole volume was finally adjusted to 100 ml with deionized water, and the mixture was adequately stirred to give a uniform solution.

The prepared composition was tested and analyzed. Table XIV provides the analytical results of the permanent wave composition in a preferred embodiment of the present invention.

TABLE XIV

| Analysis of Composition | |
|---|---|
| Thiolactic Acid Amount | 1.01M |
| Free Alkali Amount | 0.54N |
| Monoethanolamine Amount (Pretreated Product HPLC) | 0.83M |
| pH / 25°C | 9.10 |
| Molar Concentration Ratio (1): (Monoethanolamine moieties) / (Thiolactate moieties) | 0.822 |
| Molar Concentration Ratio (2): (Monoethanolamine Thiolactate) / (Thiolactate) | 0.55 |

Example 21 (Comparison)

As a comparative example with the composition of Example 20, a composition shown in Table XV was prepared according to Example 20. Table XVI provides the analytical results of this composition.

TABLE XV

| Permanent Wave Composition | |
|---|---|
| Ingredients | (w/v %) |
| Ammonium Thioglycolate (containing 50% thioglycolic acid) | 18.42 |
| Monoethanolamine | 1.73 |
| Ammonium Bicarbonate | 0.70 |
| Ammonia (28% - 30%) | 1.54 |
| Turpinal SL[*1] | 0.05 |
| Anon BL[*2] | 0.80 |

*1: Chelating Agent obtained from Henkel

*2: Lauryl Betain obtained from Nippon Oil & Fats Co., Ltd.

TABLE XV   (continued)

| Permanent Wave Composition | |
|---|---|
| Ingredients | (w/v %) |
| EMALEX 720[*4] | 0.50 |
| Polymer JR-400[*3] | 0.20 |
| Fragrance | 0.10 |
| Deionized Water | q.s. to 100mL |

*3: Cationic cellulose obtained from Union Carbide

*4: Polyoxyethylene (20 E.O.) laulylether obtained from NIHON EMULSION CO., LTD.


TABLE XVI

| Analysis of Composition | |
|---|---|
| Thioglycolic Acid Amount | 1.02 M |
| Free Alkali Amount | 0.63 N |
| pH / 25°C | 9.10 |


Examples 22 to 31 Evaluation

The neutralizing solution used for neutralization and fixing after treatment of hair with the compositions of Examples 20 and 21 was the formulation of the following Table XVII.

TABLE XVII

| Neutralizing Solution | |
|---|---|
| Ingredients | (w/v %) |
| Sodium Bromate | 5.00 |
| Disodium Hydrogenphosphate | 0.10 |
| Potassium Dihydrogen phosphate | 0.10 |
| TORAY Silicon SM 8702C[*1] | 5.00 |
| Antiseptic | Appropriate Amount |
| Deionized Water | q.s. to 100mL |

*1: Obtained from Toray - Dowcorning Corp.

On the aqueous permanent wave compositions described in Table XIII and Table XV, wave effect and the damage of hair were evaluated according to the above test and evaluation methods. The treatment with permanent wave composition was carried out at 30°C for 5 minutes, 10 minutes, 15 minutes, 20 minutes and 30 minutes, and all the neutralization treatments were carried out at 30°C for 20 minutes.

The results are shown in Table XVIII and Table XIX, respectively.

EP 0 712 623 B1

TABLE XVIII

Waving Effect and Hair Damage in Example 20

| Example No. | Evaluation / Primary Agent Treatment Time | Wave Effect | | Hair Damage |
|---|---|---|---|---|
| | | Strength : Length of Curl (mm) | Elasticity : Length of Curl (mm) | Moisture absorbency (%) |
| 22 | 5 Minutes | 11.2 | 50.1 | 25.6 |
| 23 | 10 Minutes | 8.5 | 42.2 | 38.8 |
| 24 | 15 Minutes | 7.9 | 37.8 | 43.9 |
| 25 | 20 Minutes | 7.7 | 38.3 | 44.4 |
| 26 | 30 Minutes | 7.7 | 39.1 | 46.3 |

TABLE XIX

Waving Effect and Hair Damage in Example 21 (Comparison)

| Example No. | Primary Agent Treatment Time | Wave Effect | | Hair Damage |
| --- | --- | --- | --- | --- |
| | Evaluation | Strength : Length of Curl (mm) | Elasticity : Length of Curl (mm) | Moisture absorbency (%) |
| 27 | 5 Minutes | 9.9 | 47.5 | 42.2 |
| 28 | 10 Minutes | 8.4 | 41.3 | 46.9 |
| 29 | 15 Minutes | 7.9 | 43.5 | 54.4 |
| 30 | 20 Minutes | 7.8 | 47.0 | 58.7 |
| 31 | 30 Minutes | 7.5 | 53.5 | 63.9 |

As to the permanent wave composition of the present invention according to Example 20, after the treatment time of 10 to 15 minutes (Exs. 23, 24, 25 and 26), the waving effect showed a almost fixed value, and although the damage of hair progressed in a slight degree, the progress was slow. This demonstrates that an ideal permanent waving agent could be provided wherein in over-process which is liable to occur actually in beauty salons, there is only extremely a small danger of excessive waving and hair damage. On the other hand, as to the permanent wave composition according to Example 21 (comparison), it is understood that after the treatment time of 10 to 15 minutes (Ex. Nos. 28, 29, 30 and 31), on the waving effect, particularly hair coil length is rather lengthened and hair damage fairly progresses. This means that in the case of permanent waving agents containing thioglycolic acid as the main ingredient, particularly those having a pH of >9.0, optimum time (about 10 minutes in this case) on permanent waving treatment

EP 0 712 623 B1

time is extremely narrow, and in actual treatment in beauty salons, a careful treatment such as hair check or test curl is required.

Example 32

In order to demonstrate the efficacy of the aqueous permanent wave composition of the present invention on previously damaged hair, an aqueous permanent wave composition according to the present invention was prepared and tested. In this experiment, the permanent wave formulation detailed in Table XX was employed as representative of an aqueous permanent wave composition for previously damaged hair.

TABLE XX

| Permanent Wave Composition for Previously Damaged Hair | |
|---|---|
| Ingredients | (w/v %) |
| Ammonium Thiolactate (containing 60% thiolactic acid) | 6.38 |
| Monoethanolamine Thiolactate (containing 40% thiolactic acid) | 11.69 |
| Diammonium Dithiolactate (containing 40% thiolactic acid) | 10.00 |
| Monoethanolamine | 1.50 |
| Ammonium Bicarbonate | 2.50 |
| Turpinal SL[*1] | 0.10 |
| Laurylamidopropylbetaine (35%) | 1.00 |
| Glycerol | 5.00 |
| EMALEX 520[*2] | 0.50 |
| Fragrance | 0.15 |
| Deionized Water | q.s. to 100mL |

*1: Chelating Agent obtained from Henkel

*2: Polyoxyethylene (20 E.O.) oleylether obtained from NIHON EMULSION CO., LTD.

In preparing this composition, 8 ingredients starting from the top described in Table XX were added to deionized water and dissolved uniformly. Separately, the 9th ingredient was heated and melted, the 10th ingredient was added and dissolved uniformly, and the solution was added to 30 parts deionized water to dissolve it uniformly. This solution was added to the above solution, finally the whole volume was adjusted to 100 ml with the residual part of deionized water, and the mixture was stirred uniformly.

The prepared composition was tested and analyzed. Table XXI provides the analytical results of the composition.

TABLE XXI

| Analysis of Composition | |
|---|---|
| Thiolactic Acid Amount | 0.85 M |
| Dithiodilactic Acid Amount | 4.03 w/v % |
| Free Alkali Amount | 0.56 N |
| Molar Concentration Ratio (1) | 0.808 |
| Molar Concentration Ratio (2) | 0.55 |

80 ml of this aqueous composition was applied to the whole hair of a person, after capping the hair was left as such for 10 minutes applying a dryer thereto, the hair was washed with water for one minute, 100 ml of the neutralizer used in Examples 20 and 21 was sprinkled on the hair, the hair was left as it such for 15 minutes, and finally, the rods were taken off, and the hair was washed with water and towel-dried. As to the finished hair, there was elastic force in the wave, and the hair had no permanent wave odors, was glossy and was smooth waved hair.

Claims

1. An aqueous composition for use in permanent waving of hair, comprising a reducing agent and an alkali agent, wherein

A. the composition comprises as the reducing agent thiolactic acid in the free form, or thiolactic acid in the form of a salt with at least one base chosen from ammonia, monoethanolamine and isopropanolamine, and comprises as the alkali agent at least one basic compound which is chosen from ammonium hydroxide, monoethanolamine and isopropanolamine, wherein the alkali agent is in the free form and/or forms a salt with the thiolactic acid, and

B. the molar ratio of the total monoethanolamine and/or isopropanolamine moiety to the total thiolactate moiety in the composition is from 0.3 to 1.0.

2.  A composition as claimed in claim 1, wherein the total molar concentration of the thiolactate and free thiolactic acid, when present, is from 0.3 to 2.2.

3.  A composition as claimed in claim 1 or 2, wherein the pH of the composition is from 7.5 to 9.5.

4.  A composition as claimed in any one of claims 1 to 3, wherein the molar ratio of monoethanolamine thiolactate to ammonium thiolactate is 0.25:0.75 ~ 1.0:0.0.

5.  A composition as claimed in any preceding 5 claim, wherein the molar ratio of the total monoethanolamine and/or isopropanolamine moiety to the total thiolactate moiety is from 0.5 to 1.0.

6.  A composition as claimed in any preceding claim, wherein the molar concentration of the thiolactate and thiolactic acid, when present, is from 0.4 to 1.1.

7.  A composition as claimed in any preceding claim which further comprises at least one further reducing agent chosen from ammonium thioglycolate, monoethanolamine thioglycolate, cysteine or salts thereof or N-acetyl cysteine.

8.  A composition as claimed in any preceding claim, which further comprises at least one further alkali agent chosen from guanidine, ammonium carbonate, ammonium bicarbonate, 2-amino-2-methylpropanol, lysine, arginine and sodium hydroxide.

9.  A composition as claimed in any preceding claim, which further comprises dithiodilactic acid as an oxidised derivative of thiolactic acid, or a salt thereof.

10. The use of a composition according to any preceding claim for permanent waving of hair.


**Patentansprüche**

1.  Wässrige Zusammensetzung zur Verwendung bei einer Haar-Dauerwelle, umfassend ein Reduktionsmittel und ein alkalisches Mittel, wobei

    A. die Zusammensetzung als Reduktionsmittel Thiomilchsäure in der freien Form oder Thiomilchsäure in der Form eines Salzes mit wenigstens einer aus Ammoniak, Monoethanolamin und Isopropanolamin ausgewählten Base umfasst und als das alkalisches Mittel wenigstens eine basische Verbindung, die aus Ammoniumhydroxid, Monoethanolamin und Isopropanolamin gewählt ist, wobei das Alkalimittel in der freien Form vorliegt und/oder ein Salz mit der Thiomilchsäure bildet, und

    B. das Molverhältnis der gesamten Monoethanolamin- und/oder Isopropanolaminfunktionalität zur gesamten Thiolactatfunktionalität in der Zusammensetzung von 0,3 bis 1,0 beträgt.

2.  Zusammensetzung gemäß Anspruch 1, worin die molare Gesamtkonzentration des Thiolactats und der freien Thiomilchsäure, wenn vorliegend, von 0,3 bis 2,2 ist.

3.  Zusammensetzung gemäß Anspruch 1 oder 2, worin der pH der Zusammensetzung von 7,5 bis 9,5 ist.

4.  Zusammensetzung gemäß einem der Ansprüche 1 bis 3, worin das Molverhältnis von Monoethanolaminthiolactat zu Ammoniumthiolactat 0,25:0,75 ~ 1,0:0,0 ist.

5. Zusammensetzung gemäß einem der vorhergehenden Ansprüche, worin das Molverhältnis der gesamten Monoethanolamin- und/oder Isopropanolaminfunktionalität zur gesamten Thiolactatfunktionalität von 0,5 bis 1,0 ist.

6. Zusammensetzung gemäß einem der vorhergehenden Ansprüche, worin die molare Konzentration des Thiolactats und der Thiomilchsäure, wenn vorliegend, von 0,4 bis 1,1 ist.

7. Zusammensetzung gemäß einem der vorhergehenden Ansprüche, die ferner wenigstens ein weiteres Reduktionsmittel umfasst, das aus Ammoniumthioglykolat, Monoethanolaminthioglykolat, Cystein oder Salzen davon oder N-Acetylcystein ausgewählt ist.

8. Zusammensetzung gemäß einem der vorhergehenden Ansprüche, ferner umfassend wenigstens ein weiteres alkalisches Mittel, ausgewählt aus Guanidin, Ammoniumcarbonat, Ammoniumbicarbonat, 2-Amino-2-methylpropanol, Lysin, Arginin und Natriumhydroxid.

9. Zusammensetzung gemäß einem der vorhergehenden Ansprüche, ferner Dithiodimilchsäure als ein oxidiertes Derivat der Thiomilchsäure oder ein Salz davon umfassend.

10. Verwendung einer Zusammensetzung gemäß einem der vorhergehenden Ansprüche zum Dauerwellen von Haar.

## Revendications

1. Composition aqueuse destinée à être utilisée pour l'ondulation permanente des cheveux, comprenant un agent réducteur et un agent alcalin, sachant que :

   A. la composition comprend, comme agent réducteur, l'acide thiolactique sous sa forme libre, ou l'acide thiolactique sous la forme d'un sel avec au moins une base choisie parmi l'ammoniac, la monoéthanolamine et l'isopropanolamine, et comprend, comme agent alcalin, au moins un composé basique qui est choisi parmi l'hydroxyde d'ammonium, la monoéthanolamine et l'isopropanolamine, l'agent alcalin se trouvant sous sa forme libre ou sous la forme d'un sel avec l'acide thiolactique, et
   B. le rapport molaire de la totalité des fragments de monoéthanolamine et/ou d'isopropanolamine à la totalité des fragments thiolactate dans la composition se situe entre 0,3 et 1,0.

2. Composition selon la revendication 1, dans laquelle la concentration molaire totale de thiolactate et d'acide thiolactique libre, quand il est présent, va de 0,3 à 2,2.

3. Composition selon la revendication 1 ou 2, dans laquelle le pH de la composition va de 7,5 à 9,5.

4. Composition selon l'une quelconque des revendications 1 à 3, dans laquelle le rapport molaire du thiolactate de monoéthanolamine au thiolactate d'ammonium va de 0,25:0,75 à 1,0:0,0.

5. Composition selon l'une quelconque des revendications précédentes, dans laquelle le rapport molaire de la totalité des fragments de monoéthanolamine et/ou d'isopropanolamine à la totalité des fragments thiolactate va de 0,5 à 1,0.

6. Composition selon l'une quelconque des revendications précédentes, dans laquelle la concentration molaire de thiolactate et d'acide thiolactique, quand il est présent, va de 0,4 à 1,1.

7. Composition selon l'une quelconque des revendications précédentes, qui comprend en outre au moins un autre agent réducteur choisi parmi le thioglycolate d'ammonium, le thioglycolate de monoéthanolamine, la cystéine ou ses sels, ou la N-acétylcystéine.

8. Composition selon l'une quelconque des revendications précédentes, qui comprend en outre au moins un autre agent alcalin choisi parmi la guanidine, le carbonate d'ammonium, le bicarbonate d'ammonium, le 2-amino-2-méthylpropanol, la lysine, l'arginine et l'hydroxyde de sodium.

9. Composition selon l'une quelconque des revendications précédentes, qui comprend en outre l'acide dithiolactique comme dérivé oxydé de l'acide thiolactique ou un sel de ce dernier.

**10.** Utilisation d'une composition selon l'une quelconque des précédentes revendications, pour l'ondulation permanente des cheveux.